# EUROPEAN PATENT SPECIFICATION

(11) **EP 1 758 983 B1**
(45) Date of publication and mention of the grant of the patent: **24.06.2009**
(21) Application number: 05742294.1
(22) Date of filing: 04.05.2005
(51) Int. Cl.: C12N 1/19

(54) **MUTANT SACCHAROMYCES CEREVISIAE STRAIN UTILIZING XYLOSE FOR ETHANOL PRODUCTION**
XYLOSE FÜR DIE ETHANOLPRODUKTION VERBRAUCHENDER MUTANTER SACCHAROMYCES-CEREVISIAE-STAMM
SOUCHE MUTANTE DE SACCHAROMYCES CEREVISIAE UTILISANT UN XYLOSE POUR PRODUIRE DE L'ETHANOL

(30) Priority: 07.05.2004 SE 0401191
(43) Date of publication of application: 07.03.2007
(73) Proprietor: Scandinavian Technology Group AB, 223 70 Lund (SE)
(72) Inventor: KARHUMAA, Kaisa, S-226 45 Lund (SE); GORWA-GRAUSLUND, Marie-Françoise, S-211 27 Malmö (SE)
(74) Representative: Egeröd, Lisbeth
(86) International application number: PCT/SE2005/000660
(87) International publication number: WO 2005/108552

(56) References cited:
- TRAFF-BJERRE K.L. ET AL: 'Endogenous NADPH-dependent aldose reductase activity influences product formation during xylose consumption in recombinant Saccharomyces cerevisiae.' YEAST. vol. 21, 30 January 2004, pages 141 - 150, XP002991613
- JOHANSSON BJOERN ET AL: 'The non-oxydative pentose phosphate pathway controls the fermentation rate of xylulose but not of xylose in Saccharomyces cerevisiae TMB3001.' FEMS YEAST RESEARCH. vol. 2, 2002, pages 277 - 282, XP002966585
- TRAEFF K.L. ET AL: 'Deletion of the GRE3 Aldose Reductase Gene and its Influence on Xylose Metabolism in Recombinant Strains of Saccharomyces cerevisiae Expressing the xyIA and XKS1 Genes.' APPLIED AND ENVIRONMENTAL MICROBIOLOGY. vol. 67, no. 12, December 2001, pages 5668 - 5674, XP002312912
- KIM M.D. ET AL: 'Comparison of xylitol production in recombinant Saccharomyces cerevisiae strains harboring XYL1 gene of Pichia stipitis and GRE3 gene of S. cerevisiae.' ENZYME AND MICROBIAL TECHNOLOGY. 2002, pages 862 - 866, XP002991614
- LOENN A. ET AL: 'Xylose isomerase activity influences xylose fermentation with recombinant Saccharomyces cerevisiae strains expressing mutated xyIa from Thermus thermophilus.' ENZXME AND MICROBIAL TECHNOLOGY. vol. 32, 2003, pages 567 - 573, XP002312914

## Description

### Technical field

The present invention relates to a new *Saccharomyces cerevisiae* strain having improved properties of fermenting xylose to ethanol.

### Background of the invention

Ethanol is efficiently produced from hexoses by *Saccharomyces cerevisiae*, but recombinant *S*. *cerevisiae* strains capable of xylose utilisation are needed to expand the substrate range to lignocellulosic hydrolysate. By integration of the *Pichia stipitis XYL1* and *XYL2* genes encoding xylose reductase (XR) and xylitol dehydrogenase (XDH) and the endogenous *XKS1* gene encoding xylulokinase (XK), stable xylose fermenting *S*. *cerevisiae* strains have been obtained. However, xylitol is secreted as a result of the difference in cofactor usage between the NAD(P)H-dependent XR and the strictly NAD⁺-dependent XDH steps.

Because of its dual cofactor utilization, XR from *P. stipitis* has been preferred for the construction of xylose-fermenting recombinant *S*. *cerevisiae* strains. Presently no known XR can reduce xylose to xylitol with NADH as sole cofactor. Among natural xylose-utilising yeasts *Candida utilis* XR exclusively uses NADPH, whereas *C. shehatae, P. segobiensis, P. stipitis* and *Pachysolen tannophilus* XRs use both NADPH and NADH in the reduction of xylose to xylitol. The *C. parapsilosis* XR also uses both NADPH and NADH for xylose reduction, but unlike the other yeasts it prefers NADH. Only yeasts harbouring a NADH-linked xylose reductase activity display significant alcoholic fermentation.

The ethanol yield in recombinant xylose-fermenting *S*. *cerevisiae* strains is far from the theoretical maximum of 0.51 g g⁻¹, partly because a significant fraction of the consumed xylose is secreted as xylitol. Xylitol formation in *P. tannophilus* has been reduced by addition of hydrogen acceptors. These compounds reoxidized NAD⁺, which is needed in the XDH reaction. Xylitol formation in recombinant *S*. *cerevisiae* has been reduced by the addition of acetoin, furfural and acetaldehyde. Xylitol formation can also be decreased by shifting the cofactor usage in the XR-step from NADPH to NADH. This was achieved by changing the intracellular pool of NADPH by blocking or reducing the oxidative pentose phosphate pathway (PPP) flux through modification of the glucose 6-phosphate dehydrogenase activity. This resulted in improved ethanol yield at the expense of impaired growth rate on glucose and decreased xylose consumption rate.

The *XYL1* gene has been subjected to site-specific mutagenesis to reduce the XR-affinity for NADPH. Chemical modification studies on XR showed that cysteine and histidine residues might be involved in NADPH binding. However, mutation of three cysteine residues (Cys19, Cys27 and Cys130) to serine enhanced the apparent Km for NADPH less than 4-fold, indicating that none of these cysteine residues were directly involved in NADPH binding. A 17 times lower affinity for NADPH was achieved when the *XYL1* gene carried the K270M (lysine → methionine) mutation, whereas the affinity for NADH remained unchanged. The only difference between NADPH and NADH is the presence of a phosphate group in NADPH, and it was suggested that Lys270 binds to the phosphate group of NADPH.

Attempts have also been made to change the cofactor specificity of XDH towards NADP⁺. A NADP⁺ recognition sequence from *Thermoanaerobium brockii* was introduced in the *XYL2* gene resulting in equal apparent Km values for NAD⁺ and NADP⁺. However, this was achieved at the expense of reduced NAD⁺ specificity combined with unaltered NADP⁺ specificity. The mutated *XYL2* gene mediated xylose growth when co expressed with the *XYL1* gene in *S*. *cerevisiae* while ethanolic fermentation of xylose was not reported.

Another route is to up-regulate the xylose isomerase (XI) gene and the gene for xylose reductase (XR).

### Summary of the present invention

The present invention relates to a novel *Saccharomyces cerevisiae* strains expressing xylose isomerase (XI) and xylulokinase (XK) and having been mutated by adaptation to provide improved ethanol producing yields, when consuming xylose for ethanol formation.

### Strain construction

Plasmid YEplacHXT-XI carrying the gene for *Thermus thermophilus* xylose isomerase (XI) was removed from strain TMB 3050 (DMSZ 15834) by prolonged cultivation in YPD medium. Clones lacking the plasmid were identified by replica plating on mineral medium lacking uracil. Uracil-auxotrophic clones were purified by repeated plating and one of these was named TMB 3051. TMB 3051 was transformed with plasmid pY7 (Walfridsson et al. 1997 Appl Microbiol Biotechnol 48(2):218-24), carrying the genes for xylose reductase (XR) and xylulose dehydrogenase (XDH). A control strain was made by transforming the strain TMB 3044 (precursor of TMB 3050) with pY7.

With this procedure was created a strain with all the modifications of TMB 3050 (XK and non-oxidative PPP overexpression, *GRE3* deletion, adaptation) but carrying XR and XDH instead of XI.

### Materials and methods

**Transformation.** Standard molecular biology techniques were used {Sambrook, 1989}. The lithium acetate method was used for yeast transformation {Gietz, et al 1995}.

**Cultivation conditions.** Liquid cultures of *S*. *cerevisiae* were grown in YPD medium (20 g/I peptone, 10 g/l yeast extract and 20 g/I glucose) or defined mineral medium {Verduyn, 1990 #68}, supplemented with 20 g/l glucose or 50 g/I xylose as carbon source and buffered with phthalate (10.21 g/I phthalate, 2.1 g/I KOH, pH 5.5) before sterilization. The xylose used (Acros organics, New Jersey, USA) contained 0,5-1% glucose impurity equivalent to 0,5 -1 g/I glucose in the medium when 50 g/l xylose was used. Plate cultures were done using YPD-agar or SC-plates (6.7 g/l Difco Yeast Nitrogen Base, 30 g/I agar). When required, uracil was added at concentration 40 µg/ml.

Pre-cultures for aerobic growth experiments with were cultivated until late exponential phase in defined mineral medium {Verduyn, 1990 #68} with 50 g/l xylose or 20 g/I glucose. The cells were washed with sterile water and inoculated at starting OD₆₂₀ of about 0,1 in in defined mineral medium {Verduyn, 1990 #68} with 50 g/I xylose. 50 ml cultures were grown in 500 ml baffled shake flasks and incubated at 30°C with 130 rpm shaking. All cultivations were repeated at least twice. The growth rates on xylose were determined after the impurity glucose, determined by HPLC, had been consumed.

### Results

**Aerobic growth on xylose.** TMB 3055 grew aerobically in shake-flask cultures with defined mineral medium supplied with 50 g/l xylose as the sole carbon source with growth rate of 0.16 ± 0.01 1/h.

However, the growth of the control strain (TMB 3044), carrying the same plasmid and not having the mutation acquired by the adaptation process, was in the same range.

### Oxygen limited fermentation.

In oxygen limited batch fermentations, xylose was consumed with rate 0.048 ± 0.024 g xylose/ g cells / h and ethanol was produced with yield 0.32 ± 0.10. (Table 1).

**Table 1. Oxygen limited batch fermentations with strain TMB 3055**

| | *q_{xylose}* | *Yₑₜₕₒₙₒₗ* | *Y_{xylitol}* | *Y_{glycerol}* | *Y_{acetate}* |
|---|---|---|---|---|---|
| | *(g xylose*/*g cells*/*h)* | *(g* / *g xylose)* | *(g* / *g xylose)* | *(g*/*g xylose)* | *(g* / *g xylose)* |
| TMB 3055 | 0.048 ± 0.024 | 0.32 ± 0.10 | 0.26 ± 0.03 | 0.08 ± 0.02 | 0.03 ± 0.006 |

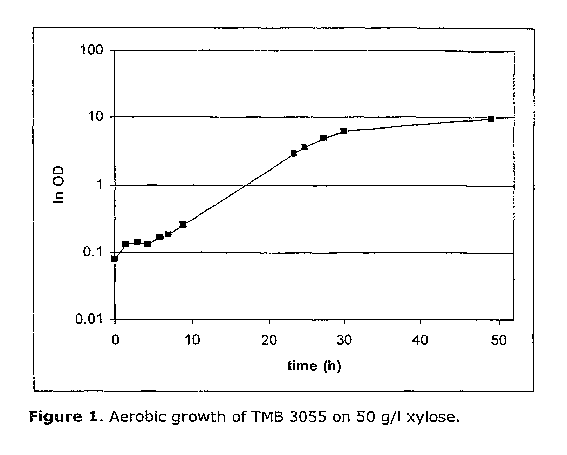

## Claims

1. A xylose utilizing *Saccharomyces cerevisiae* strain being able to utilize xylose for ethanol production, which strain is up-regulated with regard to the genes for xylose reduuctase (XR) obtained from *Pichia stipitis,* and xylulose dehydrogenase (XDH) obtained from *P. stipitis,* as well as xylulokinase(XK) obtained from *Saccharomyces cerevisiae*, said xylose reductase, and xylulose dehydrogenase being expressed on plasmid, and overexpressing the non-oxidative pentoses phosphate pathway (PPP) and comprising a deletion of the gene GRE3, as well as the strain has been adapted to xylose feeding, whereby the *XVL1* gene producing XR has been subjected to site-specific mutagenesis to reduce the XR-affinity for NADPH, whereby mutation of three cysteine residues in the positions Cys19, Cys27 and Cys130 to serine was obtained.

## Patentansprüche

1. Ein Xylose nutzender Stamm von *Saccharomyces cerevisiae*, der fähig ist, Xylose zur Ethanolproduktion zu nutzen, wobei dieser Stamm in Bezug auf die Gene für Xylosereduktase (XR), gewonnen aus *Pichia stipilis*, und Xylulosedehydrogenase (XDH), gewonnen aus *P. stipitis*, sowie Xylulokinase (XK), gewonnen aus *Saccharomyces cerevisiae* hochreguliert ist, wobei die Xylosereduktase und Xylulosedehydrogenase auf Plasmid exprimiert werden, und der den nichtoxidativen Pentosephosphatweg (PPP) überexprimiert und eine Deletion des Gens GRE3 umfasst, und der Stamm ferner an Xylosefeeding angepasst worden ist, wobei das XR produzierende XYL1 Gen einer positionsspezifischen Mutagenese unterzogen worden ist, um die XR-Affinität für NADPH zu verringern, wobei die Mutation von drei Cysteinresten in den Positionen Cys19, Cys27 und Cys130 an Serin erhalten wurde.

## Revendications

1. Souche de *Saccharomyces cerevisiae* utilisant le xylose capable d'utiliser le xylose pour la production d'éthanol, laquelle souche est régulée positivement concernant les gènes de la xylose réductase (XR) obtenu à partir de *Pichia stipitis*, et de la xylulose déshydrogénase (XDII) obtenu à partir de *P. stipitis*, ainsi que de la xylulokinase (XK) obtenu à partir de *Saccharomyces cerevisiae*, lesdites xylose réductase et xylulose déshydrogénase étant exprimées sur un plasmide, et surexprimant la voie des pentoses-phosphates non oxydative (PPP) et comprenant une délétion du gène GRE3, et la souche a été adaptée à l'alimentation avec du xylose, de sorte que le gène *XYL*1 produisant XR a été soumis à une mutagenèse spécifique de site pour réduire l'affinité de XR pour le NADPH, de sorte qu'une mutation de trois résidus cystéine aux positions Cys19, Cys27 et Cys130 en sérine a été obtenue.
